# EUROPÄISCHE PATENTSCHRIFT

(11) **EP 0 688 547 B1**
(45) Veröffentlichungstag und Bekanntmachung des Hinweises auf die Patenterteilung: **08.03.2000**
(21) Anmeldenummer: 95109362.4
(22) Anmeldetag: 16.06.1995
(51) Int. Cl.: A61F 2/52

(54) **Brustprothese für brustamputierte Frauen und Verfahren zu ihrer Herstellung**
Mammary prosthesis for women having an amputated breast and method for manufacturing the same
Prothèse mammaire pour femmes avec un sein amputé et sa procédé de fabrication

(30) Priorität: 20.06.1994 DE 4421516
(43) Veröffentlichungstag der Anmeldung: 27.12.1995
(73) Patentinhaber: F + E Gesellschaft für Bekleidungsinnovation mbH & Co. KG, 83098 Brannenburg (DE)
(72) Erfinder: Weber-Unger, Georg, A-6330 Kufstein (AT); Volk, Stephan, D-83714 Miesbach (DE)
(74) Vertreter: Haug, Dietmar, Dipl.-Ing.

(56) Entgegenhaltungen:
- WO-A-89/05615
- WO-A-94/24964
- DE-A- 2 827 076
- DE-U- 8 701 925
- GB-A- 484 367

## Beschreibung

Die Erfindung betrifft eine Brustprothese für brustamputierte Frauen, mit einem weich-elastischen Kunststoffkörper, der aus zwei längs ihres gemeinsamen Randes miteinander verbundenen Kunststoffolien und einer zwischen die Kunststoffolien hohlraumfrei eingeschlossenen Kunststoffmasse besteht, wobei der Kunststoffkörper eine der Form der natürlichen Brust nachgebildete Vorderseite und eine dem Oberkörper der Trägerin zuwendbare Rückseite hat, und mit einem die Rückseite des Kunststoffkörpers bedeckenden Stoffteil, das an dem Kunststoffkörper befestigt ist. Die Erfindung betrifft auch ein Verfahren zur Herstellung einer Brustprothese.

Eine derartige Brustprothese ist aus der DE 28 27 077 A1 bekannt. Bei dieser Brustprothese ist die auf der Rückseite des Kunststoffkörpers angeordnete Kunststoffolie mit einem Stoffteil in Form eines Gewebes, Gewirks oder Flors beschichtet. Das Stoffteil besteht aus einem Baumwollmaterial, das schweißabsorbierend ist. Das Stoffteil und die Kunststoffolie sind ganzflächig miteinander verbunden, so daß zwischen der Kunststoffolie und dem Stoffteil kein Hohlraum vorhanden ist, in den die vom Stoffteil aufgenommene Feuchtigkeit wieder austreten könnte. Infolgedessen wird das Stoffteil bei länger andauernder Schweißabsorption zunehmend feuchter, wodurch ein unerwünschtes Kleben der Prothese an der Haut der Trägerin verursacht wird und sich die Prothese aufgrund von Verdunstungskälte unangenehm kalt auf der Haut der Trägerin anfühlt.

Die beschriebenen Nachteile, die eine Folge der Beschichtung der auf der Rückseite des Kunststoffkörpers angeordneten Kunststoffolie mit einem Stoffteil sind, treten bei einer Prothese, die lose in einer sie vollständig umhüllenden Stofftasche angeordnet ist, nicht auf. Eine solche Prothese ist beispielsweise aus der US-PS-4,795,464 bekannt. Diese Prothese weist auf ihrer Rückseite eine Vertiefung auf, die durch den rückwärtigen Teil einer die Prothese umhüllenden Stofftasche, die aus einem elastischen Baumwollmaterial besteht, überspannt wird. Der die Rückseite der Stofftasche bildende Stoffteil der Tasche liegt an der Haut der Trägerin an und kann absorbierten Schweiß in die Vertiefung abgeben, so daß ein Feuchtigkeitsstau in dem auf der Haut der Trägerin anliegenden Stoffteil vermieden wird. Der Abtransport von Feuchtigkeit aus dem Stoffteil wird noch dadurch gefördert, daß sich der Kunststoffkörper relativ zu dem Stoffteil bewegen kann, wodurch eine Pumpwirkung zwischen dem Kunststoffkörper und dem Stoffteil entsteht. Die Stofftasche hat jedoch den Nachteil, daß ihre Herstellung vergleichsweise hohe Kosten verursacht und sich die Prothese relativ zur Stofftasche beim Tragen verschieben kann.

Die Aufgabe der Erfindung besteht darin, die gattungsgemäße Brustprothese so auszubilden, daß in das Stoffteil eingetretener Hautschweiß rasch aus ihm wieder abgeführt wird, wobei die Prothese ohne eine Stofftasche verwendet werden kann.

Die Aufgabe der Erfindung wird dadurch gelöst, daß das Stoffteil nur mit dem Rand der Kunststoffolien fest verbunden ist.

Bei der erfindungsgemäßen Brustprothese besteht also im innerhalb des Prothesenrandes liegenden Bereich keine feste Verbindung zwischen dem Stoffteil und der auf der Rückseite des Kunststoffkörpers angeordneten Kunststoffolie. Von dem Stoffteil aufgenommener Hautschweiß kann somit in den Bereich zwischen dem Stoffteil und der Rückseite des Kunststoffkörpers abgeführt werden. Infolgedessen kann sich Feuchtigkeit nicht mehr in dem Stoffteil stauen, wodurch seine Neigung zum Kleben an der Haut der Trägerin vermindert ist und sich das Stoffteil stets warm auf der Haut anfühlt. Der Bereich zwischen dem Stoffteil und der Rückseite des Kunststoffkörpers kann ein einzelner Hohlraum sein, wenn die Rückseite des Kunststoffkörpers mit einer Vertiefung versehen ist, wie sie bei den an sich bekannten Schalenprothesen vorzufinden ist. Sind in der Vertiefung in an sich bekannter Weise Versteifungen angeordnet, ergeben sie mehrere Kammern zwischen dem Stoffteil und der Rückseite des Kunststoffkörpers. Ist die Rückseite des Kunststoffkörpers hingegen eben ausgebildet, wie es bei den sogenannten Vollprothesen der Fall ist, liegt im unverformten Zustand des Kunststoffkörpers und des Stoffteils das Stoffteil lose an der Rückseite des Kunststoffkörpers an. Da sich jedoch die Prothese beim Tragen durch Bewegungen der Trägerin elastisch verformt, bleibt das Stoffteil nicht ständig an der Rückseite des Kunststoffkörpers liegen sondern hebt sich von der Rückseite des Kunststoffkörpers mehr oder minder stark entsprechend der elastischen Verformung der Prothese ab, so daß in dem Bereich zwischen dem Stoffteil und der Rückseite des Kunststoffkörpers ein Raum oder Räume variabler Größe entstehen. Die Abgabe von Feuchtigkeit aus dem Stoffteil in den Bereich zwischen dem Stoffteil und der Rückseite des Kunststoffkörpers ist bei der erfindungsgemäßen Prothese immer möglich, ganz gleich ob nun das Stoffteil an der Rückseite des Kunststoffkörpers lose anliegt oder sich zwischen dem Stoffteil und der Rückseite des Kunststoffkörpers ein oder mehrere Hohlräume befinden. Hohlräume zwischen dem Stoffteil und der Rückseite des Kunststoffkörpers der erfindungsgemäßen Prothese vergrößern die Aufnahmekapazität für Feuchtigkeit in dem Bereich zwischen dem Stoffteil und der Rückseite des Kunststoffkörpers und fördern die Luftzirkulation in diesem Bereich, so daß die Verdunstung der darin befindlichen Feuchtigkeit eventuell schneller vonstatten geht. Die erfindungsgemäße Brustprothese ist kostengünstiger in der Herstellung als eine vergleichbare Prothese mit einer Prothesentasche aus Stoff, weil bei der erfindungsgemäßen Prothese auf eine Prothesentasche verzichtet werden kann, die wegen der aufwendigeren Fertigung und des größeren Materialbedarfs teurer als das Stoffteil der erfindungsgemäßen Prothese ist. Die erfindungsgemäße Prothese vereinigt die Vorteile einer Prothese mit Prothesentasche und einer Prothese, deren Rückseite mit einem textilen Material beschichtet ist in sich, ohne die Nachteile dieser Prothesen in Kauf nehmen zu müssen. Infolgedessen zeichnet sich die erfindungsgemäße Prothese durch einen außerordentlich hohen Tragekomfort aus.

Das Stoffteil kann aus einem thermoplastischen Material bestehen und ist dann mit dem Rand der Kunststoffolien verschweißt. Auf diese Weise wird das Stoffteil fest und dauerhaft mit dem Kunststoffkörper verbunden, wobei die Befestigung des Stoffteils an dem Kunststoffkörper durch Schweißen besonders dann von Vorteil ist, wenn auch die beiden Kunststoffolien miteinander verschweißt sind, denn es kann ein- und dieselbe Technik beim Verbinden der Kunststoffolien und bei der Befestigung des Stoffteils an dem Kunststoffkörper eingesetzt werden. Das Verschweißen des Stoffteils mit den Kunststofffolien bedeutet nicht zwangsläufig, daß das Stoffteil längs der Schweißlinie mit den Kunststoffolien vollständig verschmilzt. Unter dem Begriff "Verschweißen" soll auch ein Befestigungsverfahren verstanden werden, bei dem das Stoffteil nur an seiner Oberfläche mit den Kunststoffolien verschmilzt.

Anstatt das Stoffteil durch Schweißen mit dem Kunststoffkörper zu verbinden, kann das Stoffteil auch an den Rand der Kunststoffolien angeklebt werden. Auch das Kleben führt zu einer festen und dauerhaften Verbindung zwischen dem Stoffteil und dem Kunststoffkörper, und die Befestigung des Stoffteils an dem Kunststoffkörper durch Kleben ist immer dann angebracht, wenn sich das für das Stoffteil gewählte Material nicht oder weniger gut zum Schweißen eignet, beispielsweise, wenn das Stoffteil ganz oder teilweise aus einer Naturfaser wie Baumwolle, besteht.

Die feste Verbindung zwischen dem Stoffteil und dem Kunststoffkörper ermöglicht es, den Rand des Stoffteils genau bündig mit dem Rand der Kunststoffolien zu machen. Vorzugsweise hat daher das Stoffteil einen mit dem Rand der Kunststoffolien abschließenden Rand, und ist der Rand des Stoffteils mit dem Rand der Kunststoffolien fest verbunden. Im Gegensatz dazu ragt eine Prothesentasche immer über den Prothesenrand etwas hinaus, weil sich längs des Prothesenrandes ein Saum erstreckt, der den vorderen Teil und den hinteren Teil der Prothesentasche miteinander verbindet. Ein überstehender Stoffrand ist jedoch unerwünscht, wenn die Gefahr besteht, daß er aus dem Bekleidungsstück herausschaut, in welchem die Prothese getragen wird.

Die feste Verbindung zwischen dem Stoffteil und dem Kunststoffkörper eröffnet aber auch die Möglichkeit, die Prothese mittels des Stoffteils in dem Bekleidungsstück zu fixieren. Es wird daher vorgeschlagen, daß das Stoffteil einen wenigstens abschnittsweise über den Rand der Kunststoffolien hinausstehenden Randbereich hat, der mittels einer an ihm befestigten Haltevorrichtung an einem Büstenhalter oder dem Oberteil eines Badebekleidungsstücks befestigbar ist, in dem die Prothese tragbar ist. Beispielsweise kann die Haltevorrichtung aus einem oder mehreren Klettverschlußteilen bestehen, deren Gegenstück an dem Kleidungsstück befestigt ist.

Für das Stoffteil kann eine Vielzahl verschiedener Materialien verwendet werden. Die wesentlichen Kriterien bei der Auswahl des für das Stoffteil zu verwendenden Materials sind seine Fähigkeit, leicht am Kunststoffkörper befestigt werden zu können, eine gute Dehnbarkeit, eine hohe Reißfestigkeit, ein gutes Feuchtigkeitsaufnahme- und -abgabevermögen, eine angenehme Weichheit und die Fähigkeit, leicht gereinigt werden zu können. Wenn das Stoffteil an den Kunststoffkörper angeschweißt werden soll, ist von es Vorteil, wenn es aus Polyamid- oder Polyesterfasern hergestellt ist. Wegen seiner bekannten hautfreundlichen Eigenschaften kann das Stoffteil auch aus Baumwolle oder aus einer Mischung aus Polyamid- und/oder Polyesterfasern und Baumwolle bestehen. Wenn das Stoffteil ganz aus Baumwolle besteht, wird es vorzugsweise durch Kleben an dem Kunststoffkörper befestigt. Steht eine hohe Dehnbarkeit des Stoffteils im Vordergrund, z.B. dann, wenn es sich bei Teilamputationen an das stehengebliebene Brustgewebe anschmiegen soll, ohne auf die Operationsnarbe zu drücken, ist es von Vorteil, wenn das Stoffteil einen Faseranteil aus Polyester und/oder Polymidfasern und einen Faseranteil aus Elastofasern aufweist. Soll das Stoffteil besonders gut Feuchtigkeit von der Hautoberfläche abtransportieren, ist es von Vorteil, wenn das Stoffteil aus einem Gewebe aus Microfasern besteht, die Feuchtigkeit in Dampfform transportieren können. Besonders geeignet sind dann solche feinstfibrilligen Microfasern, die aus Polyester oder Polyamid bestehen. Derartige Microfaser-Gewebe sind dem Fachmann auch unter der Bezeichnung "Klimastoffe" bekannt, weil sie den dampfförmigen Körperschweiß durch das Gewebe hindurch verdunsten lassen.

Die Erfindung schafft auch ein Verfahren zur Herstellung einer Brustprothese für brustamputierte Frauen, bei dem ein eine Einfüllöffnung aufweisender Beutel aus zwei aufeinanderliegenden Polyurethanfolien durch Verschweißen der beiden Folien längs einer den späteren Prothesenrand bildenden Linie hergestellt wird, eine unvernetzte Silikonkautschukmasse in den Beutel eingefüllt wird, während der gefüllte Beutel in einer zweiteiligen Form angeordnet ist, die eine Aushöhlung hat, deren Form der der natürlichen Brust nachgebildeten Vorderseite der Prothese entspricht, und ein Stoffteil an der Rückseite der Prothese befestigt wird und die Silikonkautschukmasse unter Wärmezufuhr vernetzt wird. Erfindungsgemäß ist dieses Verfahren dadurch gekennzeichnet, daß das Stoffteil aus einem thermoplastischen Material hergestellt wird und das Stoffteil und die Polyurethanfolien beim Verschweißen der beiden Folien miteinander verschweißt werden, derart, daß das Stoffteil nur am späteren Prothesenrand befestigt ist.

Das erfindungsgemäße Verfahren hat den Vorteil, daß die Befestigung des Stoffteils an den Polyrethanfolien gemeinsam mit dem Verschweißen der Polyurethanfolien erfolgen kann. Es ist also nur ein Schweißvorgang erforderlich, bei dem gleichzeitig die beiden Polyurethanfolien und das Stoffteil längs des späteren Prothesenrandes miteinander verschweißt werden. Der auf diese Weise hergestellte Beutel wird dann mit dem Stoffteil nach oben weisend in der Form angeordnet und mit der unvernetzten Silikonkautschukmasse befüllt. In bekannter Weise wird dann die zweiteilige Form geschlossen und in einen Wärmeschrank eingebracht, in welchem die Vernetzung der Silikonkautschukmasse unter Wärmezufuhr erfolgt. Nachdem die Silikonkautschukmasse vernetzt ist, wird die zweiteilige Form aus dem Wärmeschrank herausgenommen und geöffnet. Das aus Folienbeutel, vernetzter Silikonkautschukmasse und angeschweißtem Stoffteil bestehende Formteil, dessen Form der späteren Prothese weitgehend entspricht, wird der zweiteiligen Form entnommen und der Endbearbeitung zugeführt, bei der insbesondere der noch überstehende Randbereich der Polyurethanfolien und des Stoffteils bis zur Schweißnaht abgeschnitten wird, die dann den Rand der fertigen Prothese darstellt.

Ein Ausführungsbeispiel der Erfindung ist in der Zeichnung dargestellt und wird im folgenden näher beschrieben. Es zeigt
- Fig. 1: einen Querschnitt durch eine erfindungsgemäße Brustprothese, die als Vollprothese ausgebildet ist,
- Fig. 2: einen Querschnitt durch eine erfindungsgemäße Brustprothese, bei der die Rückseite des Kunststoffkörpers eine Vertiefung aufweist,
- Fig. 3: einen Querschnitt durch eine erfindungsgemäße Brustprothese, bei der die Rückseite des Kunststoffkörpers eine Vertiefung aufweist und in der Vertiefung ein Polsterkörper angeordnet ist, und
- Fig. 4: einen Querschnitt durch eine erfindungsgemäße Brustprothese, bei der die Rückseite des Kunststoffkörpers eine Vertiefung aufweist und das Stoffteil einen über den Rand des Kunststoffkörpers vorstehenden Randabschnitt zur Befestigung an einem Bekleidungsstück hat.

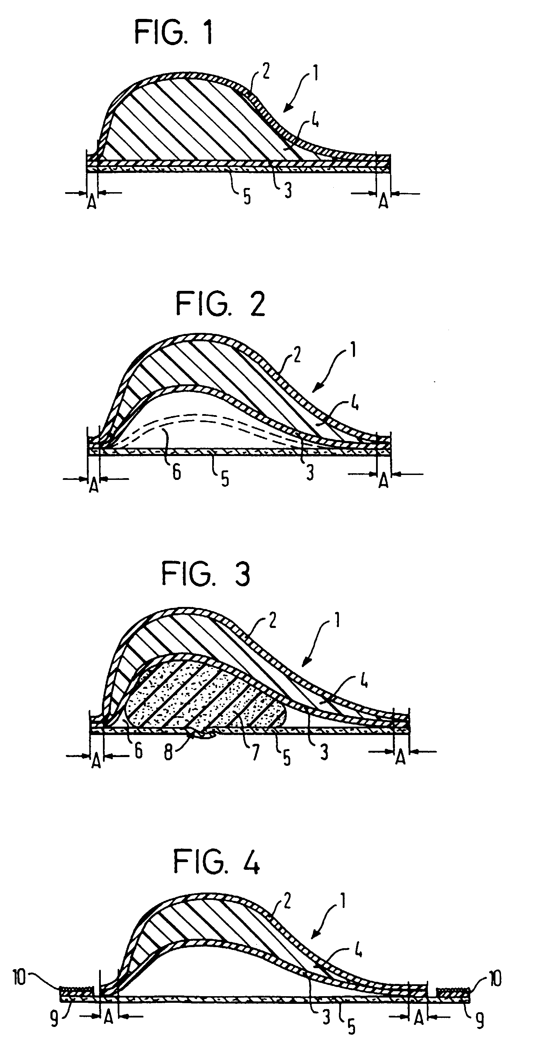

Zunächst wird auf Fig. 1 Bezug genommen. Die in Fig. 1 dargestellte Brustprothese weist einen weich-elastischen Kunststoffkörper 1 auf, der aus zwei längs ihres gemeinsamen Randes, im Bereich A miteinander verschweißten Polyurethanfolien 2, 3 und einer zwischen die Polyurethanfolien hohlraumfrei eingeschlossenen additionsvernetzten Zweikomponenten-Silikonkautschukmasse 4 besteht. Die mit der Ziffer 2 bezeichnete Polyurethanfolie begrenzt den Kunststoffkörper 1 an seiner Vorderseite, deren Form der natürlichen Brust nachgebildet ist. Die mit der Ziffer 3 bezeichnete Polyurethanfolie ist an der Rückseite des Kunststoffkörpers 1 angeordnet, die dem Körper der Prothesenträgerin zugekehrt ist. Ein Stoffteil 5 bedeckt die Rückseite des Kunststoffkörpers 1 und ist im Bereich A, in welchem die Polyurethanfolien 2, 3 miteinander verschweißt sind, an dem Kunststoffkörper 1 durch Verschweißen mit den Kunststoffolien 2, 3 befestigt. Das Stoffteil 5 ist also nur mit dem Rand der Kunststoffolien 2, 3 fest verbunden und hat in dem innerhalb des Bereichs A liegenden Bereich keine feste Verbindung mit dem Kunststoffkörper 1. Bei der in Fig. 1 dargestellten Brustprothese ist die Rückseite des Kunststoffkörpers 1 eben ausgebildet. Infolgedessen wird diese Brustprothese auch als Vollprothese bezeichnet. Bei einer solchen Prothese liegt das Stoffteil 5 in dem innerhalb des Bereichs A liegenden Bereich lose an der Polyurethanfolie 3 an. Wenn sich die Brustprothese beim Tragen elastisch verformt, z.B. schwingt, können sich zwischen dem Stoffteil 5 und der Polyurethanfolie 3 Kammern ausbilden, deren Größe durch die Größe der Verformung der Prothese bestimmt wird. Diese Kammern können durch das Stoffteil 5 hindurch Luft aufnehmen und abgeben, wodurch der Körperbereich, in dem die Prothese anliegt, angenehm belüftet wird. Die Belüftung wiederum wirkt der Hautschweißbildung in diesem Kammerbereich entgegen. Der trotz Belüftung entstehende Hautschweiß wird von dem Stoffteil 5 aufgenommen und in den Bereich zwischen der Polyurethanfolie 3 und dem Stoffteil abgeführt. Es bleibt somit kein kühlender Schweiß auf der Haut zurück.

Bei den in den Fig. 2, 3 und 4 dargestellten Brustprothesen werden die gleichen Bezugszeichen für die gleichen oder die sich entsprechenden Teile, die auch bei der in Fig. 1 dargestellten Brustprothese vorhanden sind, verwendet.

Die in Fig. 2 dargestellte Brustprothese unterscheidet sich von der in Fig. 1 dargestellten Brustprothese dadurch, daß auf der Rückseite des Kunststoffkörpers 1 eine Vertiefung 6 vorgesehen ist. Die auf der Rückseite des Kunststoffkörpers 1 angeordnete Polyurethanfolie 3 folgt der Kontur der Vertiefung 6. Eine solche Brustprothese wird üblicherweise als Schalenprothese bezeichnet. Die Polyurethanfolien 2, 3 sind wieder im Bereich A miteinander und mit dem Stoffteil 5 verschweißt. Im Unterschied zu der in Fig. 1 dargestellten Brustprothese liegt das Stoffteil 5 in dem innerhalb des Bereichs A liegenden Bereich nicht lose an der Polyurethanfolie 3 an sondern sie überbrückt die Vertiefung 6. Da das Stoffteil weich und elastisch ist, kann es sich leicht an die Form des bei einer Teilamputation stehengelassenen Brustgewebes anpassen, wobei es dann in die Vertiefung 6 hineinragt, wie durch die gestrichelte Doppellinie in Fig. 2 angedeutet ist. Auch in verformtem Zustand des Stoffteils 5 entsteht oder verbleibt im Bereich zwischen dem Stoffteil 5 und der Polyurethanfolie 3 ein Zwischenraum, in den und aus dem Luft strömen kann, so daß stets für eine gute Belüftung des Körperbereichs gesorgt ist, in dem die Prothese anliegt. Dadurch, daß dieser Zwischenraum meistens wesentlich größer ist als der, der sich zwischen dem Stoffteil 5 und der Polyurethanfolie 3 bei der in Fig. 1 dargestellten Prothese ausbilden kann, ist der Luftaustausch bei der in Fig. 2 dargestellten Prothese besser als bei der in Fig. 1 dargestellten Prothese. Aufgrund des verbesserten Luftaustausches bei der in Fig. 2 dargestellten Prothese kann die von dem Stoffteil 5 aufgenommene und in den Zwischenraum zwischen dem Stoffteil 5 und der Polyurethanfolie 3 abgegebene Feuchtigkeit rascher verdunsten, so daß das Feuchtigkeitsaufnahme- und -abgabevermögen des Stoffteils 5 der in Fig. 2 dargestellten Prothese noch besser als bei der in Fig. 1 dargestellten Prothese ist.

Bei der in Fig. 3 dargestellten Brustprothese hat der Kunststoffkörper 1 eine ähnliche Vertiefung 6 wie der Kunststoffkörper der in Fig. 2 dargestellten Brustprothese. Um zu vermeiden, daß eine solche Prothese, besonders wenn sie sehr groß ist und die Vertiefung 6 aus Gewichtsgründen sehr ausgeprägt ist, unter ihrem Eigengewicht zusammensackt, hat die in Fig. 3 dargestellte Prothese einen Polsterkörper 7 in der Vertiefung 6, wobei der Polsterkörper 7 zwischen dem Stoffteil 5 und der Polyurethanfolie 3 angeordnet ist. Der Polsterkörper 7 kann beispielsweise aus einem Schaumkunststoff oder einem Faserknäuel bestehen, der von einem nicht dargestellten Beutel umhüllt sein kann. Auch bei der in Fig. 3 dargestellten Brustprothese ist das Stoffteil 5 nur im Bereich A mit den Kunststoffolien 2, 3 verschweißt. In dem innerhalb des Bereichs A liegenden Bereich hat das Stoffteil 5 keine feste Verbindung zu dem Kunststoffkörper 1. An dem Polsterkörper 7 liegt das Stoffteil 5 lose an. Das Stoffteil 5 hat eine schlitzförmige Öffnung 8 die durch zwei sich überlappende Abschnitte des Stoffteils 5 gebildet ist. Durch diese Öffnung 8 kann der Polsterkörper 7 herausgenommen und in die Vertiefung 6 eingesetzt werden. Ähnlich wie bei der in Fig. 2 dargestellten Brustprothese sorgt das Stoffteil 5 bei der in Fig. 3 dargestellten Brustprothese für eine gute Belüftung und Schweißableitung in dem Körperbereich, in dem die Prothese anliegt.

In Fig. 4 ist eine ähnliche Brustprothese wie in Fig. 2 dargestellt. Die in Fig. 4 dargestellte Brustprothese unterscheidet sich von der in Fig. 2 dargestellten Brustprothese nur dadurch, daß das Stoffteil 5 einen Randabschnitt 9 aufweist, der über den durch den Rand der Polyurethanfolien 2, 3 gebildeten Prothesenrand hinausragt. An diesem Randabschnitt 9 ist ein Klettverschlußteil 10 befestigt, dessen nicht dargestelltes Gegenstück an dem Büstenhalter oder dem Oberteil eines Badebekleidungsstücks befestigt ist, in dem die Prothese getragen wird. Durch Zusammendrücken des Klettverschlußteils 10 und des nicht dargestellten Gegenstücks kann die Prothese an dem Bekleidungsstück gegen unerwünschtes Verrutschen oder Verdrehen gesichert werden. Wie bei den in den Figuren 1 bis 3 dargestellten Brustprothesen ist das Stoffteil 5 der in Fig. 4 dargestellten Brustprothese nur im Bereich A mit dem Kunststoffkörper 1 durch Verschweißen mit dem Rand der Polyurethanfolien 2, 3 fest verbunden. Die Belüftungs- und Feuchtigkeitstransporteigenschaften des Stoffteils 5 der in Fig. 4 dargestellten Brustprothese sind die gleichen wie bei der in Fig. 2 dargestellten Brustprothese.

Ein sehr geeignetes Material für das Stoffteil 5 ist ein aus Microfasern hergestellter Stoff mit der Warenbezeichnung "Ninfea", der von der Firma Jersey Lomellina SPA, Via Don Pedrinelli, 94, I-24030 Carvico (BG), Italien, erhältlich ist und aus 75 % Nylon® und 25 % Elastan® besteht.

Die Herstellung der erfindungsgemäßen Brustprothese erfolgt in der Weise, daß die beiden Polyurethanfolien und das Stoffteil aufeinandergelegt und längs einer den späteren Prothesenrand bildenden Linie bis auf eine später zu schließende Einfüllöffnung miteinander verschweißt werden, so daß ein Beutel entsteht. Der weitere Verfahrensablauf ist insoweit, als er die Prothesen gemäß Fig. 1 und 3 betrifft, Stand der Technik und wurde bereits eingangs beschrieben. Nur auf die Endbearbeitung der in den Fig. 1 und 3 dargestellten Prothesen soll noch verwiesen werden, denn bei der Endbearbeitung dieser Prothesen wird der über den späteren Prothesenrand noch hinausstehende Randabschnitt des Stoffteils zusammen mit dem über den späteren Prothesenrand noch hinausstehende Randabschnitt der Polyurethanfolien abgeschnitten. Sowohl bei der Befestigung des Stoffteils an den Polyurethanfolien als auch bei der Endbearbeitung der Prothese erfordert das Stoffteil somit keinen zusätzlichen Arbeitsschritt im Vergleich zum herkömmlichen Verfahren (soweit als das Verfahren die Herstellung der Prothesen gemäß Fig. 1 bis 3 betrifft). Bei der in Fig. 4 dargestellten Prothese erfolgt der Schweißvorgang wie bei den in den Fig. 1 bis 3 dargestellten Prothesen. Die Endbearbeitung der in Fig. 4 dargestellten Prothese unterscheidet sich jedoch von der Endbearbeitung der in den Fig. 1 bis 3 gezeigten Prothese dadurch, daß der über den späteren Prothesenrand hinausstehende Randabschnitt 9 des Stoffteils zumindest abschnittsweise stehengelassen wird und an dem stehengelassenen Randabschnitt das Klettverschlußteil 10 befestigt wird.

## Patentansprüche

1. Brustprothese für brustamputierte Frauen, mit einem weich-elastischen Kunststoffkörper (1), der aus zwei längs ihres gemeinsamen Randes miteinander verbundenen Kunststoffolien (2, 3) und einer zwischen die Kunststoffolien (2, 3) hohlraumfrei eingeschlossenen Kunststoffmasse (4) besteht, wobei der Kunststoffkörper (1) eine der Form der natürlichen Brust nachgebildete Vorderseite und eine dem Oberkörper der Trägerin zuwendbare Rückseite hat, und mit einem die Rückseite des Kunststoffkörpers (1) bedeckenden Stoffteil (5), das an dem Kunststoffkörper (1) befestigt ist,
**dadurch gekennzeichnet**, daß das Stoffteil (5) nur mit dem Rand der Kunststoffolien (2, 3) fest verbunden ist.

2. Brustprothese nach Anspruch 1,
**dadurch gekennzeichnet**, daß das Stoffteil (5) aus einem thermoplastischen Material besteht und mit dem Rand der Kunststoffolien (2, 3) verschweißt ist.

3. Brustprothese nach Anspruch 2,
**dadurch gekennzeichnet**, daß die Kunststoffolien (2, 3) miteinander und mit dem Stoffteil (5) mit einer einzigen Schweißnaht (A) verschweißt sind.

4. Brustprothese nach Anspruch 1,
**dadurch gekennzeichnet**, daß das Stoffteil (5) an den Rand der Kunststoffolien (2, 3) angeklebt ist.

5. Brustprothese nach einem der vorhergehenden Ansprüche,
**dadurch gekennzeichnet**, daß das Stoffteil (5) einen mit dem Rand der Kunststoffolien (2, 3) abschließenden Rand hat und der Rand des Stoffteils (5) mit dem Rand der Kunststoffolien (2, 3) fest verbunden ist.

6. Brustprothese nach einem der Ansprüche 1 bis 4,
**dadurch gekennzeichnet**, daß das Stoffteil (5) einen wenigstens abschnittsweise über den Rand der Kunststoffolien (2, 3) hinausstehenden Randbereich (9) hat, der mittels einer an ihm befestigten Haltevorrichtung (10) an einem Büstenhalter oder Oberteil eines Badebekleidungsstücks befestigbar ist, in dem die Prothese tragbar ist.

7. Brustoprothese nach Anspruch 6,
**dadurch gekennzeichnet**, daß die Haltevorrichtung aus einem oder mehreren Klettverschlußteilen (10) besteht.

8. Brustprothese nach einem der vorhergehenden Ansprüche,
**dadurch gekennzeichnet**, daß die Rückseite des Kunststoffkörpers (1) eine Vertiefung (6) aufweist und ein Polsterkörper (7) in der Vertiefung (6) angeordnet ist, und das Stoffteil (5) eine schlitzförmige Öffnung (8) hat, durch die der Polsterkörper (7) in die Vertiefung (6) einführbar und daraus entnehmbar ist.

9. Brustprothese nach einem der vorhergehenden Ansprüche,
**dadurch gekennzeichnet**, daß das Stoffteil (5) aus Polyamid- oder Polyesterfasern hergestellt ist.

10. Brustprothese nach einem der Ansprüch 1 bis 8,
**dadurch gekennzeichnet**, daß das Stoffteil (5) aus einer Mischung aus Polyamid- und/oder Polyesterfasern und Baumwolle besteht.

11. Brustprothese nach einem der Ansprüche 1 oder 4 bis 8,
**dadurch gekennzeichnet**, daß das Stoffteil (5) aus Baumwolle besteht.

12. Brustprothese nach einem der Ansprüche 1 bis 8
**dadurch gekennzeichnet**, daß das Stoffteil (5) einen Faseranteil aus Polyester- oder Polyamidfasern und einen Faseranteil aus Elastofasern aufweist.

13. Brustprothese nach einem der Ansprüche 1 bis 8,
**dadurch gekennzeichnet,** daß das Stoffteil (5) aus einem Gewebe oder Gewirk aus Microfasern besteht, die Feuchtigkeit in Dampfform transportieren können.

14. Brustprothese nach Anspruch 12,
**dadurch gekennzeichnet**, daß die Microfasern aus Polyester oder Polyamid bestehen.

15. Verfahren zur Herstellung einer Brustprothese für brustamputierte Frauen, bei dem ein eine Einfüllöffnung aufweisender Beutel aus zwei aufeinanderliegenden Polyurethanfolien (2, 3) durch Verschweißen der beiden Folien (2, 3) längs einer den späteren Prothesenrand bildenden Linie (A) hergestellt wird, eine unvernetzte Silikonkautschukmasse (4) in den Beutel eingeführt wird, während der gefüllte Beutel in einer zweiteiligen Form angeordnet ist, die eine Aushöhlung hat, deren Form der der natürlichen Brust nachgebildeten Vorderseite der Prothese entspricht, ein Stoffteil (5) an der Rückseite der Prothese befestigt wird und die Silikonkautschukmasse (4) unter Wärmezufuhr vernetzt wird,
**dadurch gekennzeichnet**, daß das Stoffteil (5) aus einem thermoplastischen Material hergestellt wird und das Stoffteil (5) und die Polyurethanfolien (2, 3) beim Verschweißen der beiden Folien (2, 3) miteinander verschweißt werden, derart, daß das Stoffteil (5) nur am späteren Prothesenrand befestigt ist.

16. Verfahren nach Anspruch 15,
**dadurch gekennzeichnet**, daß die Polyurethanfolien (2, 3) und das Stoffteil (5) einen über die Schweißlinie (A) hinausstehenden Randbereich haben, der in einem dem Entformen folgenden Endbearbeitungsschritt bis auf die Schweißlinie (A) abgeschnitten wird.

## Claims

1. A breast prosthesis for mastectomy patients, comprising a soft elastic plastic member (1) consisting of two plastic sheets (2, 3) joined along their common edge and a plastic material (4) enclosed without cavities between the sheets (2, 3), the front of the plastic member (1) simulating the shape of the natural breast and the back being adapted to face the upper part of the wearer's body, the prosthesis also comprising a cloth part (5) covering the back of the plastic member (1) and secured thereto, characterised in that the cloth part (5) is firmly connected only to the edge of the plastic sheets (2, 3).

2. A breast prosthesis according to claim 1,
characterised in that the cloth part (5) consists of a thermoplastic material and is welded to the edge of the plastic sheets (2, 3).

3. A breast prosthesis according to claim 2,
characterised in that the plastic sheets (2, 3) are welded to one another and to the cloth part (5) by a single seam (A).

4. A breast prosthesis according to claim 1,
characterised in that the cloth part (5) is stuck to the edge of the plastic sheets (2, 3).

5. A breast prosthesis according to any one of the preceding claims, characterised in that the cloth part (5) has an edge which is flush with the edge of the plastic sheets (2, 3), and the edge of the cloth part (5) is firmly connected to the edge of the plastic sheets (2, 3).

6. A breast prosthesis according to any one of claims 1 to 4, characterised in that the cloth part (5) has an edge zone (9) which projects at least at some places beyond the edge of the plastic sheets (2, 3) and has an attached retaining means (10) for securing to a brassiere or the upper part of a bathing costume in which the prosthesis can be worn.

7. A breast prosthesis according to claim 6,
characterised in that the retaining means comprises one or more hook-and-loop-fastener parts (10).

8. A breast prosthesis according to any one of the preceding claims, characterised in that the back of the plastic member (1) has a recess (6) and a pad member (7) is disposed in the recess (6), and the cloth part (5) has an slot opening (8) through which the pad member (7) can be inserted into or removed from the recess (6).

9. A breast prosthesis according to any one of the preceding claims, characterised in that the cloth part (5) is made of polyamide or polyester fibres.

10. A breast prosthesis according to any one of claims 1 to 8, characterised in that the cloth part (5) consists of a mixture of polyamide and/or polyester fibres and cotton.

11. A breast prosthesis according to any one of claims 1 or 4 to 8, characterised in that the cloth part (5) is of cotton.

12. A breast prosthesis according to any one of claims 1 to 8, characterised in that the cloth part (5) has a polyester or polyamide fibre component and an elastomeric fibre component.

13. A breast prosthesis according to any one of claims 1 to 8, characterised in that the cloth part (5) comprises a woven or knitted fabric of microfibres which can convey moisture in vapour form.

14. A breast prosthesis according to claim 12, characterised in that the microfibres are of polyester or polyamide.

15. A method of manufacturing a breast prosthesis for mastectomy patients, wherein a bag having an opening for filling is made from two superposed polyurethane sheets (2, 3) by welding the two sheets (2, 3) along a line (A) forming the subsequent edge of the prosthesis, a non-crosslinked silicone rubber material (4) is inserted into the bag and the bag after being filled is placed in a two-part mould having a cavity corresponding in shape to the front of the prosthesis which simulates the natural breast, a cloth part (5) is secured to the back of the prosthesis and the silicone rubber material (4) is cross-linked by heating, characterised in that the cloth part (5) is made of thermoplastic material and the cloth part (5) and the polyurethane sheets (2, 3), when the two sheets (2, 3) are welded together, are welded in such a way that the cloth part (5) is secured only to the subsequent edge of the prosthesis.

16. A method according to claim 15, characterised in that the polyurethane sheets (2, 3) and the cloth part (5) have an edge zone which projects beyond the weld line (A) and is cut down to the weld line (A) in a final processing step after removal from the mould.

## Revendications

1. Prothèse mammaire pour des femmes amputées du sein, avec un corps de matière plastique (1) élastique et souple, composé de deux feuilles de matière plastique (2, 3), assemblées entre elles le long de leur bord commun, et d'une masse de matière plastique (4), incluse sans espace vide entre les feuilles de matière plastique (2, 3), le corps de matière plastique (1) présentant un côté avant imitant la forme du sein naturel et un côté arrière pouvant être tourné vers le buste de la porteuse, et avec une pièce de tissu (5) recouvrant le côté arrière du corps de matière plastique (1), qui est fixée sur le corps (1), **caractérisée en ce que** la pièce de tissu (5) n'est assemblée fixement qu'avec le bord des feuilles de matière plastique (2, 3).

2. Prothèse mammaire suivant la revendication 1, caractérisée en ce que la pièce de tissu (5) se compose d'une matière thermoplastique et est soudée avec le bord des feuilles de matière plastique (2, 3).

3. Prothèse mammaire suivant la revendication 2, caractérisée en ce que les feuilles de matière plastique (2, 3) sont soudées entre elles et avec la pièce de tissu (5) par un joint de soudure (A) unique.

4. Prothèse mammaire suivant la revendication 1, caractérisée en ce que la pièce de tissu (5) est collée sur le bord des feuilles de matière plastique (2, 3).

5. Prothèse mammaire suivant l'une des revendications précédentes, caractérisée en ce que la pièce de tissu (5) a un bord se terminant avec le bord des feuilles de matière plastique (2, 3), et le bord de la pièce de tissu (5) est assemblé fixement avec le bord des feuilles de matière plastique (2, 3).

6. Prothèse mammaire suivant l'une des revendications 1 à 4, caractérisée en ce que la pièce de tissu (5) présente une zone de bordure (9), dépassant au moins par sections du bord des feuilles de matière plastique (2, 3) et qui peut être fixée au moyen d'un dispositif de retenue (10), fixé sur elle, sur un soutien-gorge ou un haut de maillot de bain, dans lequel peut être portée la prothèse.

7. Prothèse mammaire suivant la revendication 6, caractérisée en ce que le dispositif de retenue se compose d'une ou de plusieurs fermetures de type Velcro (10).

8. Prothèse mammaire suivant l'une des revendications précédentes, caractérisée en ce que le côté arrière du corps de matière plastique (1) présente une cavité (6) et un corps de rembourrage (7) est disposé dans la cavité (6), et la pièce de tissu (5) présente un orifice (8) en forme de fente, au travers duquel le corps de rembourrage (7) peut être introduit dans la cavité (6) et en être retiré.

9. Prothèse mammaire suivant l'une des revendications précédentes, caractérisée en ce que la pièce de tissu (5) est réalisée en fibres de polyamide ou de polyester.

10. Prothèse mammaire suivant l'une des revendications 1 à 8, caractérisée en ce que la pièce de tissu (5) se compose d'un mélange de fibres de polyamide et/ou polyester et de coton.

11. Prothèse mammaire suivant l'une des revendications 1 et 4 à 8, caractérisée en ce que la pièce de tissu (5) se compose de coton.

12. Prothèse mammaire suivant l'une des revendications 1 à 8, caractérisée en ce que la pièce de tissu (5) présente une part de fibres de polyester ou polyamide et une part d'élastofibres.

13. Prothèse mammaire suivant l'une des revendications 1 à 8, caractérisée en ce que la pièce de tissu (5) se compose d'un tissu ou d'un tissu à mailles en microfibres, qui peuvent transporter de l'humidité en forme de vapeur.

14. Prothèse mammaire suivant la revendication 12, caractérisée en ce que les microfibres se composent de polyester ou de polyamide.

15. Procédé de fabrication d'une prothèse mammaire pour des femmes amputées du sein, dans lequel un sachet, présentant un orifice de remplissage, est fabriqué à partir de deux feuilles de polyuréthanne (2, 3) superposées, par soudage des deux feuilles (2, 3) le long d'une ligne (A) formant le bord ultérieur de la prothèse, une masse de caoutchouc silicone (4) non réticulée est introduite dans le sachet, tandis que le sachet rempli est disposé dans un moule en deux parties doté d'une cavité, dont la forme correspond au côté avant de la prothèse imitant le sein naturel, une pièce de tissu (5) est fixée sur le côté arrière de la prothèse et la masse de caoutchouc silicone (4) est réticulée sous apport de chaleur, **caractérisé en ce que** la pièce de tissu (5) est réalisée en une matière thermoplastique, et la pièce de tissu (5) et les feuilles de polyuréthanne (2, 3) sont soudées entre elles lors du soudage des deux feuilles (2, 3), de sorte que la pièce de tissu (5) n'est fixée que sur le bord ultérieur de la prothèse.

16. Procédé suivant la revendication 15, caractérisé en ce que les feuilles de polyuréthanne (2, 3) et la pièce de tissu (5) présentent une zone de bordure, dépassant de la ligne de soudage (A) et découpée, dans une phase de traitement final suivant le démoulage, jusqu'à la ligne de soudage (A).
